(19) Europäisches Patentamt
European Patent Office
Office européen des brevets

(11) Publication number: 0 272 742 B1

(12) **EUROPEAN PATENT SPECIFICATION**

(45) Date of publication of patent specification: **16.10.91** (51) Int. Cl.[5]: **C07C 323/62**

(21) Application number: **87202490.6**

(22) Date of filing: **11.12.87**

# (54) Method for preparation of mercaptobenzoates.

(30) Priority: **12.12.86 US 940997**
**12.12.86 US 940995**

(43) Date of publication of application:
**29.06.88 Bulletin 88/26**

(45) Publication of the grant of the patent:
**16.10.91 Bulletin 91/42**

(84) Designated Contracting States:
**AT BE CH DE ES FR GB GR IT LI LU NL SE**

(56) References cited:
**US-A- 3 867 433**
**US-A- 4 692 545**
**US-A- 4 704 467**

**HOUBEN-WEYL: "Methoden der Organischen Chemie", vol. IX, 4th edition, 1955, page 113, Georg Thieme Verlag, Stuttgart, DE**

(73) Proprietor: **STAUFFER AGRICULTURAL CHEMICALS COMPANY, INC.**
**c/0 ICI Americas Inc. Concord Pike & New Murphy Road**
**Wilmington Delaware 19897(US)**

(72) Inventor: **Carter, Charles Garvie**
**9524 Bruce Drive**
**Silver Spring Maryland 20901(US)**
Inventor: **Wehrenberg, Peter Karl**
**3937 Greenwood Avenue**
**Oakland California 94602(US)**

(74) Representative: **Smulders, Theodorus A.H.J., Ir.**
**Vereenigde Octrooibureaux Nieuwe Parklaan 107**
**NL-2587 BP Den Haag(NL)**

EP 0 272 742 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Compounds of the type

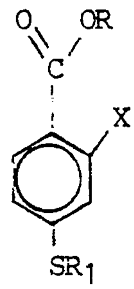

wherein R is alkyl, $R_1$ is alkyl, alkenyl, aryl or benzyl and X is halogen or nitro, are useful intermediates in the synthesis of pesticides, such as herbicides whose structures include alkylthio and alkylsulfonyl benzoates. These intermediates have been produced by various methods, including the displacement of a halogenated benzoic acid by a mercaptan in the presence of a base in an alcohol solvent to give a mercaptobenzoic acid, as described in U.S. Patent 3,867,433. These methods are characterized by low yields or are limited to benzoates that are substituted at the 3-position by a strong electron-attracting group.

### Summary of the Invention

An alkyl 4-nitro-2-substituted benzoate is reacted with a mercaptan in the presence of an inorganic base and a polar aprotic solvent or a nonpolar aprotic solvent and a phase transfer agent. The nitro group at the 4-position is selectively displaced under mild conditions to give a high yield of an alkyl 4-alkylthio-2-substituted benzoate.

When a phase transfer catalyst and a nonpolar aprotic solvent are used, the solvent is also useful in a subsequent oxidation step of the 4-alkylthio moiety. Thus the need for a change of solvent or isolation of intermediate between steps is avoided. Optionally, the solution of intermediate mercaptobenzoate can be washed with water to remove undesired salts and then oxidized without isolation of the intermediate.

### Detailed Description of the Invention

According to this invention, compounds of the formula

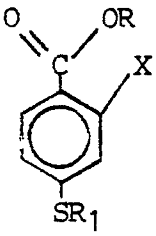

wherein R is alkyl; $R_1$ is alkyl, alkenyl, aryl or benzyl; and X is halogen or nitro, can be prepared by reacting a compound of the formula

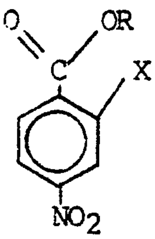

wherein R and X are as defined, with a mercaptan having the formula $R_1$-SH wherein $R_1$ is as defined in the presence of an inorganic base in (a) a nonpolar aprotic solvent which is inert to nucleophilic addition and a catalytic amount of a phase transfer catalyst which promotes formation of a soluble nucleophile and allows displacement of the 4-nitro group with a mercapto group; or (b) a polar aprotic solvent.

The process of this invention allows the selective displacement of the nitro group of an alkyl 4-nitro-2-substituted benzoate in the presence of an inorganic base and a polar aprotic solvent or a nonpolar aprotic solvent and a phase transfer catalyst to prepare an alkyl 4-alkylthio-2-substituted benzoate. The resulting benzoate can be converted to the corresponding benzoic acid via conventional methods, with an overall yield of up to 99.67% of the benzoic acid based on the starting nitrobenzoate.

Mercaptans which are useful in this reaction can be selected from alkyl, alkenyl, aryl or benzyl mercaptans. The term "alkyl" includes both straight and branched chain saturated acylic hydrocarbyl moieties and generally includes moieties having from 1 to 8 carbon atoms, preferably from 1 to 3. The term "alkenyl" includes both straight and branched chain unsaturated acyclic hydrocarbyl moieties and generally includes moieties having from 2 to 8 carbon atoms, preferably from 2 to 3. The term "aryl" includes phenyl and substituted phenyl. The inorganic bases which can be used include potassium carbonate, sodium carbonate, tripotassium phosphate and trisodium phosphate. Non-polar aprotic solvents which can be used include hydrocarbons such as benzene, toluene, xylenes; halogenated hydrocarbons such as dichloromethane, 1,2-dichloroethane, chlorobenzene; ethers such as tetrahydrofuran, diethyl ether, diisobutyl ether and other non-polar aprotic solvents. Polar aprotic solvents which can be used include acetone, methyl ethyl ketone, acetonitrile, dimethylformamide, N-methyl pyrrolidone, dimethylacetamide, methyl isobutyl ketone and other polar aprotic solvents. Phase transfer catalysts which can be used include tris-(3,7-dioxaheptyl)-amine (TDA-1), tricaprylylmethylammonium chloride (Aliquat 336®), tetra-lower alkyl substituted ammonium, phosphonium, sulfonium and sulfoxonium halides, crown ethers and criptates.

When a non-polar solvent is used, the reaction is preferably run at a temperature from about 0° to about 200° C, more preferably from about 25° to about 100° C, most preferably from about 40° to about 60° C. This reaction can be run at atmospheric, sub-atmospheric or super-atmospheric pressure, preferably at atmospheric pressure. The phase transfer catalyst can be present in from about 0.5 to about 100 mole %, preferably from about 1.0 to about 20 mole %, most preferably from about 3 to about 10 mole %. Preferred bases are potassium carbonate and sodium carbonate, most preferably potassium carbonate. Preferred non-polar solvents are hydrocarbons and halogenated hydrocarbons, more preferably halogenated hydrocarbons, most preferably chlorinated hydrocarbons such as 1,2-dichloroethane.

When a polar aprotic solvent is used, the reaction is preferably run at a temperature from about 0° to about 160° C, more preferably from about 20° to about 70° C. The reaction can be run at sub-atmospheric to super-atmospheric pressure, preferably at atmospheric pressure. Preferred bases are trisodium phosphate, tripotassium phosphate, sodium carbonate and potassium carbonate, more preferably sodium carbonate and potassium carbonate, most preferably potassium carbonate. Preferred polar aprotic solvents are acetone, methyl ethyl ketone and methyl isobutyl ketone.

When performing a 2-step reaction as in the present invention, the solvent must be compatible in each step. 1,2-Dichloroethane, although the solvent of choice of the present invention, is only one of many solvents which are suitable. It is a useful solvent in forming the mercaptobenzoate and is not subject to reaction with oxidants such as sodium hypochlorite in the oxidation reaction.

The process of this invention can be better understood by reference to the following examples. Examples 1 and 3 illustrate methods for the preparation of the mercaptobenzoate utilizing a polar aprotic solvent. Example 2 serves to confirm the structure of Example 1 through analysis of the resulting sulfonyl benzoic acid and also serves to illustrate the use of the solvent of choice of Example 1 in the oxidation step. The product of Example 1 need not be isolated, but can be further reacted before or after removal of the salts formed in Example 1. If desired, solvent can be added or removed to achieve the desired volume in the oxidation step.

The process of this invention can be better understood by reference to the following examples.

## EXAMPLE 1

### Preparation of Methyl 2-Chloro-4-methylthiobenzoate

To a multineck round bottom flask equipped with a dry ice condenser and a septum with a gas inlet were added methyl 2-chloro-4-nitrobenzoate (4.3 grams g, 0.02 moles), 40 milliliters (ml) 1,2-dichloroethane, 3.9 g potassium carbonate (0.028 m) and 0.3 g (0.001 m) TDA-1. The mixture was heated to 50° C. Methyl mercaptan was introduced in an above-surface addition until the mercaptan refluxed in the dry ice condenser. After approximately 20 minutes of reflux, high pressure liquid chromatography (HPLC) showed 92.5% of product present in the mixture.

The reaction mixture was treated with 40 ml water followed by 20 ml 1N hydrochloric acid (HCl) and the organic phase evaporated under reduced pressure to yield 4.3 g of the benzoate with 96.8 area % of the desired material shown by high pressure liquid chromatography (HPLC).

## EXAMPLE 2

### Preparation of 2-Chloro-4-methanesulfonyl benzoic acid

To a single neck, round-bottom flask were added 4.3 g (0.02 m) methyl 2-chloro-4-methylthiobenzoate produced in Example I and 60 ml 1,2-dichloroethane. Sodium hypochlorite, 85 g (0.06 m, 5.25% in water) was added dropwise and the exothermic reaction was allowed to heat. When the addition was complete, the mixture was heated to 60° C over 1.5 hours then allowed to stir overnight at ambient temperature. The excess sodium hypochlorite was decomposed with 4.5 g (0.075 mole) sodium bisulfite. 6.4 g (0. 16 mole) 50% sodium hydroxide (NaOH) were added to the mixture which was then heated to 60° C over 2 hours. The mixture was cooled to ambient temperature and extracted with 2 x 12 ml 1,2-dichloroethane (EDC). The EDC was extracted with 25 ml water and the pH of the aqueous phase was adjusted to 14 with 50% NaOH. Aqueous phases were combined and

acidified to pH 0-1 with 18% hydrochloric acid and the resulting solids filtered off to yield 3.93 g of product with a melting point of 187-192 °C. HPLC determined the sulfone to be 94.9 weight percent of the benzoic acid with a technical yield of 91.4%.

EXAMPLE 3

Preparation of Methyl 2-Chloro-4-methylthiobenzoate

To a multi-neck round bottom flask equipped as in Example I were added 21.6 g (0.1m) methyl 2-chloro-4-nitrobenzoate, 125 ml 1,2-dichloroethane, 27.6 g (0.2m) potassium carbonate, and 2.0 g (0.005 m) Aliquat® 336. The mixture was heated to 60 °C. 14.4 g (0.3 m) methyl mercaptan were introduced in an above-surface addition over a period of 6 minutes. The mixture was allowed to stir while heating at 58 °C for 12 hours. The temperature was then increased to 70 °C and the reaction allowed to stir another 7 hours. HPLC showed the reaction mixture to contain 74.2% of the desired product.

The following Examples 4 and 5 teach the use of a polar aprotic solvent.

EXAMPLE 4

Preparation of Ethyl 2-Nitro-4-methylthiobenzoate

To a 3-necked, round-bottom flask fitted with condenser and gas inlet, 104 grams (g), 0.752 mole (m) of potassium carbonate and 300 milliliters (ml) of acetone were added. While the mixture was stirring, 33.8 g (0.705 m) of methyl mercaptan was introduced via sub-surface addition followed by addition of 112.8 g (0.47 m) of ethyl 2,4-dinitrobenzoate. The mixture was stirred ten minutes and allowed to stand overnight at room temperature. The mixture was then evaporated under reduced pressure, extracted with ethyl acetate, washed with water followed by brine, dried over magnesium sulfate and evaporated to dryness under reduced pressure to yield 113.3 g (100% technical yield) of a red oil. Infrared (IR), nuclear magnetic resonance (NMR) and mass spectroscopy (MS) confirmed the structure as the desired product.

EXAMPLE 5

Preparation of Methyl 2-Chloro-4-ethylthiobenzoate

In a round-bottom flask, 15 g (0.07 m) methyl 2-chloro-4-nitrobenzoate, 13.8 g (0.1 m) potassium carbonate, 5.4 g (0.09 m) ethanethiol and 250 ml acetone were combined and allowed to stir overnight. The acetone was removed under reduced pressure, the residue extracted with dichloromethane, treated with 2 x 150 ml water to remove salts, dried over magnesium sulfate and evaporated under reduced pressure to yield 14.6 g of an oil. No analyses were performed at this stage. The product of this reaction was converted to the benzoic acid by hydrolysis with sodium hydroxide and ethanol for identification by analysis of the benzoic acid. The structure was confirmed by IR, NMR and MS.

**Claims**

1. A method of preparing a compound having the formula

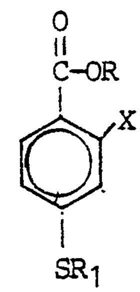

wherein R is alkyl; $R_1$ is alkyl, alkenyl, aryl or benzyl; and X is halogen or nitro comprising reacting a benzoate of the formula

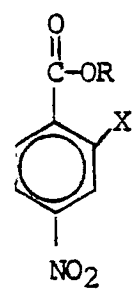

wherein R and X are as defined with a mercaptan of the formula $R_1$-SH wherein $R_1$ is as defined in the presence of an inorganic base and a polar aprotic solvent or a non-polar aprotic solvent and a phase transfer catalyst.

2. A method according to Claim 1 wherein the reaction is run in a non-polar aprotic solvent and a phase transfer catalyst.

3. A method according to Claim 1 wherein R and $R_1$ are each $C_1$-$C_3$ alkyl and X is chlorine or nitro.

4. A method according. to Claim 1 wherein the reaction is run in a polar aprotic solvent.

5. A method according to Claim 2 wherein the temperature is from about 25° to about 100° C.

6. A method according to Claim 4 wherein the temperature is from about 20° to about 70° C.

7. A method according to Claim 2 wherein the catalyst is tris-(3,7-dioxaheptyl)amine or tricaprylylmethylammonium chloride.

## Revendications

1. Un procédé de préparation d'un dérivé de formule:

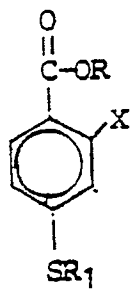

dans laquelle:
- R est un groupe alkyle;
- $R_1$ est un groupe alkyle, alkényle, aryle ou benzyle; et
- X est un atome d'halogène ou un groupe nitro consistant à faire réagir un benzoate de formule:

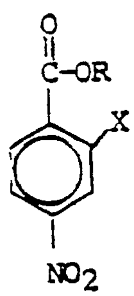

dans laquelle R et X sont tels que définis,

avec un mercaptan de formule $R_1$-SH dans laquelle $R_1$ est tel que défini, en présence d'une base inorganique et d'un solvant aprotique polaire ou d'un solvant aprotique non polaire et d'un catalyseur de transfert de phase.

2. Un procédé selon la revendication 1, dans lequel la réaction est mise en oeuvre dans un solvant aprotique non polaire et un catalyseur de transfert de phase.

3. Un procédé selon la revendication 1, dans lequel R et $R_1$ sont chacun un groupe alkyle en $C_1$-$C_3$ et X est un atome de chlore ou un groupe nitro.

4. Un procédé selon la revendication 1, dans lequel la réaction est mise en oeuvre dans un solvant aprotique polaire.

5. Un procédé selon la revendication 2, dans lequel la température est de l'ordre d'environ 25 à environ 100° C.

6. Un procédé selon la revendication 4, dans lequel la température est de l'ordre d'environ 20 à environ 70° C.

7. Un procédé selon la revendication 2, dans lequel le catalyseur est du tris-(3,7-dioxaheptyl)amine ou chlorure de tricaprylylméthylammonium.

## Patentansprüche

1. Verfahren zur Herstellung einer Verbindung der Formel:

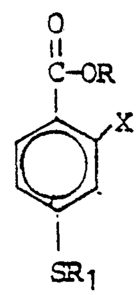

worin R Alkyl, $R_1$ Alkyl, Alkenyl, Aryl oder Benzyl und X Halogen oder Nitro bedeuten, dadurch **gekennzeichnet**, daß ein Benzoat der Formel:

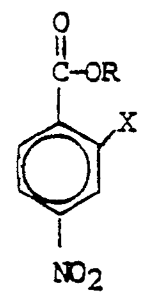

worin R und X die gegebenen Definitionen besitzen, mit einem Mercaptan der Formel $R_1$-SH, worin $R_1$ die gegebene Definition besitzt, in Anwesenheit einer anorganischen Base und

eines polaren aprotischen Lösungsmittels oder eines nichtpolaren aprotischen Lösungsmittels und eines Phasentransferkatalysators umgesetzt wird.

**2.** Verfahren nach Anspruch 1, dadurch **gekennzeichnet,** daß die Reaktion in einem nichtpolaren aprotischen Lösungsmittel und in Anwesenheit eines Phasentransferkatalysators durchgeführt wird.

**3.** Verfahren nach Anspruch 1, dadurch **gekennzeichnet,** daß R und $R_1$ je $C_1$-$C_3$-Alkyl und X Chlor oder Nitro bedeuten.

**4.** Verfahren nach Anspruch 1, dadurch **gekennzeichnet,** daß die Reaktion in einem polaren aprotischen Lösungsmittel abläuft.

**5.** Verfahren nach Anspruch 2, dadurch **gekennzeichnet,** daß die Temperatur von etwa 25 bis etwa 100° C beträgt.

**6.** Verfahren nach Anspruch 4, dadurch **gekennzeichnet,** daß die Temperatur von etwa 20 bis etwa 70° C beträgt.

**7.** Verfahren nach Anspruch 2, dadurch **gekennzeichnet,** daß der Katalysator Tris-(3,7-dioxaheptyl)-amin oder Tricaprylylmethylammoniumchlorid ist.